# EUROPEAN PATENT SPECIFICATION

(11) **EP 3 383 271 B1**
(45) Date of publication and mention of the grant of the patent: **24.08.2022**
(21) Application number: 16871705.6
(22) Date of filing: 05.12.2016
(51) Int. Cl.: A61B 5/16, A61B 5/00, A61B 5/374

(54) **SYSTEM FOR SCREENING DELIRIUM PATIENTS FOR THE PRESENCE OF ENCEPHALOPATHY**
SYSTEM ZUM SCREENING VON DELIRIUM-PATIENTEN AUF DAS VORHANDENSEIN VON ENZEPHALOPATHIE
SYSTÈME DE DÉTECTION DE PRÉSENCE D'ENCÉPHALOPATHIE CHEZ DES PATIENTS ATTEINTS DE DELIRIUM

(30) Priority: 04.12.2015 US 201562263325 P
(43) Date of publication of application: 10.10.2018
(60) Divisional application: 22185761.8
(73) Proprietor: University Of Iowa Research Foundation, Iowa City, Iowa 52242-5500 (US)
(72) Inventor: CROMWELL, John, Iowa City, Iowa 52245 (US); SHINOZAKI, Gen, Iowa City, Iowa 52246 (US)
(74) Representative: Appleyard Lees IP LLP
(86) International application number: PCT/US2016/064937
(87) International publication number: WO 2017/096358

(56) References cited:
- WO-A1-2013/142051
- WO-A1-2014/040175
- WO-A1-2014/176286
- WO-A1-2015/039689
- US-A1- 2007 032 737
- US-A1- 2014 235 969
- US-A1- 2015 099 946
- LECHINGER, JULIA ET AL.: 'CRS-R score in disorders of consciousness is strongly related to spectral EEG at rest.' JOURNAL OF NEUROLOGY vol. 260, no. 9, 2013, pages 2348 - 2356, XP055387044

## Description

### TECHNICAL FIELD

Disclosed are systems and methods for predicting, screening, and monitoring of encephalopathy / delirium, and, more specifically, to systems and methods for determining the presence, absence, or likelihood of subsequent development of encephalopathy / delirium in a patient by signal analysis.

### BACKGROUND

Encephalopathy - commonly diagnosed and known as "delirium" - is a common, under-diagnosed, and very dangerous medical condition. As discussed herein, "delirium" generally relates to the syndrome which is typically diagnosed clinically based on physicians' assessment based on diagnostic criteria based on patients' symptoms, while "encephalopathy" relates to the underlying physiological condition. As used herein, both or either "delirium" and / or "encephalopathy" may be used in conjunction with the various implementations, though the use of one is not necessarily intended to exclude the other.

Delirium - or encephalopathy - has been associated with high mortality, increased risk of developing irreversible decline in brain function, increased occurrences of preventable complications, longer hospital stays, and higher likelihood of discharge to a nursing home rather than home. These represent a serious "brain failure" condition, commonly seen in the wide variety of hospital settings including postsurgical patients as well as older general medicine patients. The mortality rate associated with delirium is approximately 40%, as high as acute myocardial infarction. At a cost of over $150 billion (USD) annually in the United States alone, delirium is a significant burden on the healthcare system in the United States, and internationally. Despite the healthcare costs and severity of complications, there is no effective approach in place to prevent and recognize delirium.

One reason for the under-recognition of delirium is a lack of simple objective measurements to identify impending development of delirium. There is no device to measure for impending delirium, such as an electrocardiogram does for impending heart attacks or a blood test for blood glucose levels to monitor for high risk of complications from diabetes.

To date, efforts to detect delirium have relied upon two major methods, both of which fall short of the practical needs of a modem hospital environment. Screening instruments, largely based upon chart review and patient interview, have been unsuccessful due to challenges implementing these into clinical workflows and providing ongoing training for healthcare providers to use such instruments. In addition, they exhibit poor sensitivity in routine use.

Electroencephalography (EEG) may effectively differentiates delirium from normal brain function, however, it is logistically impossible to use for screening of delirium as it requires a skilled technician to administer a 16- to 24-lead EEG test and a sub-specialized neurologist to interpret the study. This takes hours for each patient, and it is almost impossible to implement on large numbers of patients in busy hospital settings. In addition, EEG has not been used to predict development of delirium, only to confirm its presence.

Needs exist for improved systems and methods for predicting, screening, and monitoring of encephalopathy / delirium.

### BRIEF SUMMARY

The present invention is defined with reference to the appended claims. Discussed herein are various devices, systems and methods relating to systems, devices and methods for detecting, identifying or otherwise predicting encephalopathy or delirium in a patient. In various implementations, a device is utilized to detect diffuse slowing - a hallmark of encephalopathy.

Systems and methods are described for using various tools and procedures for predicting, screening, and monitoring of encephalopathy. The tools and procedures described herein may be used in conjunction with one or more additional tools and/or procedures for predicting, screening, and monitoring of encephalopathy. The examples described herein relate to predicting, screening, and monitoring of encephalopathy for illustrative purposes only. For multi-step processes or methods, steps may be performed by one or more different parties, servers, processors, and the like.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a system for patient delirium screening, including a handheld screening device including a housing; at least two sensors configured to record one or more brain signals and generate one or more values; a processor and at least one module configured to: perform spectral density analysis on the one or more values and output data presenting an indication of the presence, absence, or likelihood of the subsequent development of encephalopathy. Corresponding computer systems, apparatus, and computer programs recorded on one or more computer storage devices, each configured to perform the actions of the methods, may be included.

Implementations may include one or more of the following features. The system where the module is configured to compare one or more values from the one or more brain signals to a threshold. The system where the threshold is a ratio including a number of occurrences of high frequency waves to a number of occurrences of low frequency waves. The system where the one or more brain signals are electroencephalogram (EEG) signals. The system where there are two sensors. The system where the housing includes a display. The system where the processor is disposed within the housing. The system where the one or more values are selected from the group including of: high frequency waves, low frequency waves, and combinations thereof. The system where the one or more values are numeric representations of the number of occurrences of each of the one or more features over a period of time. The system where the threshold is predetermined. The system where the threshold is established on the basis of a machine learning model. The system further including a handheld housing including a display, where: the at least two sensors are in electronic communication with the housing, the processor is disposed within the housing, and the display is configured to depict the output data. The system further including a validation module configured to evaluate signal brain, where the processor converts the one or more brain frequencies into signal data, and the validation module discards the signal data that exceeds at least one pre-determined signal quality threshold. The system where the signal data is partitioned into windows of equal duration. The device further including a signal processing module. The device further including a validation module. The device further including a threshold module. Implementations of the described techniques may include hardware, a method or process, or computer software on a computer-accessible medium.

One general aspect includes a system for evaluating the presence of encephalopathy, including: a. at least two sensors configured to record one or more brain frequencies; a processor; at least one module configured to: compare brain wave frequencies over time; perform spectral density analysis on the brain wave frequencies to establish a ratio; compare the ratio against an established threshold; and output data presenting an indication of the presence, absence, or likelihood of the subsequent development of encephalopathy. This may include corresponding computer systems, apparatus, and computer programs recorded on one or more computer storage devices, each configured to perform the actions of the methods.

Implementations may include one or more of the following features. The system where the threshold is predetermined. The system where the threshold is established on the basis of a machine learning model. The system further including a handheld housing including a display, where: the at least two sensors are in electronic communication with the housing, the processor is disposed within the housing, and the display is configured to depict the output data. The system further including a validation module configured to evaluate signal brain, where the processor converts the one or more brain frequencies into signal data, and the validation module discards the signal data that exceeds at least one pre-determined signal quality threshold. The system where the signal data is partitioned into windows of equal duration. The device further including a signal processing module. The device further including a validation module. The device further including a threshold module. Implementations of the described techniques may include hardware, a method or process, or computer software on a computer-accessible medium.

One general aspect includes a handheld device evaluating the presence, absence, or likelihood of the subsequent development of encephalopathy in a patient, including: a housing; at least one sensor configured to generate at least one brain wave signal; at least one processor; at least one system memory; at least one program module configured to perform spectral density analysis on the at least one brain wave signal and generate patient output data; and a display configured to depict the patient output data. Computer systems, apparatus, and computer programs recorded on one or more computer storage devices, each configured to perform the actions of the methods, may be included.

Implementations may include one or more of the following features. The device further including a signal processing module. The device further including a validation module. The device further including a threshold module. The device further comprising a feature analysis module. The device further comprising a signal processing module. Implementations of the described techniques may include hardware, a method or process, or computer software on a computer-accessible medium.

One or more computing devices may be adapted to provide desired functionality by accessing software instructions rendered in a computer-readable form. When software is used, any suitable programming, scripting, or other type of language or combinations of languages may be used to implement the teachings contained herein. However, software need not be used exclusively, or at all. The methods and systems set forth herein may also be implemented by hard-wired logic or other circuitry, including but not limited to application-specific circuits. Combinations of computer-executed software and hard-wired logic or other circuitry may be suitable as well.

### BRIEF DESCRIPTION OF THE DRAWINGS

The accompanying drawings, which are included to provide a further understanding of the invention. In the drawings:
FIG. 1A shows an overview of an exemplary screening system in use on a patient.
FIG. 2A shows an example of diffuse slowing in brainwaves as would appear on an electroencephalogram.
FIG. 2B shows an example diffuse slowing in brainwaves as would appear on an electroencephalogram.
FIG. 3A shows an example of diffuse slowing in brainwaves as would appear on an electroencephalogram.
FIG. 3B shows an example of diffuse slowing in brainwaves as would appear on an electroencephalogram.
FIG. 3C shows an example of diffuse slowing in brainwaves as would appear on an electroencephalogram.
FIG. 4A shows an exemplary system for predicting, screening, and monitoring of delirium.
FIG. 4B shows an exemplary system for computation aspects of predicting, screening, and monitoring of delirium
FIG. 5A shows an exemplary system for the predicting, screening, and monitoring of encephalopathy.
FIG. 5B shows an exemplary system for the predicting, screening, and monitoring of encephalopathy.
FIG. 5C shows an exemplary system for the predicting, screening, and monitoring of encephalopathy.
FIG. 5D shows another exemplary system for the predicting, screening, and monitoring of encephalopathy.
FIG. 6 shows program modules and program data of a screening device, according to an exemplary embodiment.
FIG. 7 is an overview of a method for predicting, screening, and monitoring of encephalopathy, according to an exemplary embodiment.
FIG. 8A is an overview of a method for predicting, screening, and monitoring of encephalopathy, according to an exemplary embodiment.
FIG. 8B is an overview of a method for predicting, screening, and monitoring of encephalopathy, according to an exemplary embodiment.
FIG. 9A shows an example of raw electroencephalogram signals.
FIG. 9B shows an example of raw electroencephalogram signals.
FIG. 9C shows an example of spectral density analysis.
FIG. 9D shows an example of spectral density analysis.
FIG. 10 shows an example of spectral density analysis over time.
FIG. 11 shows an example of screening results at optimum frequency, according to an exemplary embodiment.
FIG. 12 shows an example of a machine learning model to identify characteristics of encephalopathy.
FIG. 13 an exemplary lift chart.

### DETAILED DESCRIPTION

Disclosed or contemplated herein are systems, methods and devices able to provide objective clinical measurements of encephalopathy the clinical form of delirium. These implementations detect the presence of diffuse slowing in the brain waves of patients - a hallmark of the onset of encephalopathy. The implementations discussed herein are able to detect diffuse slowing by performing a spectral density analysis on brain waves recorded from a small number of discrete locations on the head of the patient, thereby enabling easier bedside diagnosis, such as with a handheld device. That is, the various implementations are able to record a brain waves via two or more leads placed on the head of a patient, and execute an algorithm to evaluate the ratio of recorded low frequency to high frequency waves and compare that ratio against a determined threshold to identify the onset of encephalopathy. These implementations may utilize machine learning and additional data, such as that from medical records, to improve diagnostic accuracy.

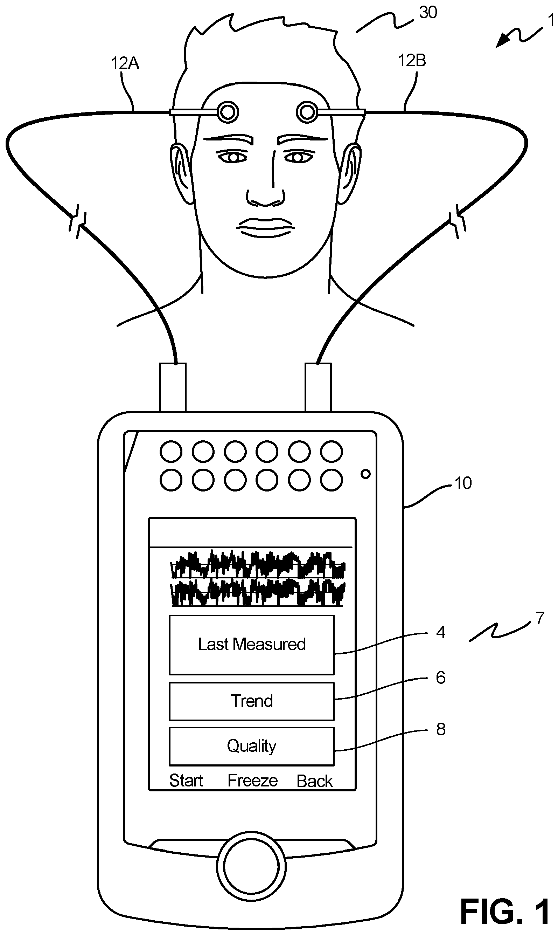

The disclosed systems, devices and methods relate to non-invasive, point of care diagnostics using fewer than the sixteen-, twenty- or twenty four-lead EEGs found in the prior art. For example, as shown generally in FIG. 1, in certain implementations a 2-lead "bispectral electroencephalography" (BSEEG) screening system 1 is employed, which can be performed with a handheld screening device 10 by applying two leads 12A, 12B to the forehead of a patient 30 for less than 10 minutes. While in these implementations, two leads or channels in the BSEEG are used for purposes of explanation, it is understood that many numbers of leads or channels are contemplated herein. In various implementations, the device 10 is able display graphical and / or numerical representations 7 of useful information for use in diagnosis of encephalopathy / delirium, such as the last measured value 4, the trend 6, signal quality 8 and the like. It is understood that these representations 7 can be the result of understood graphical user interface techniques on the display 16.

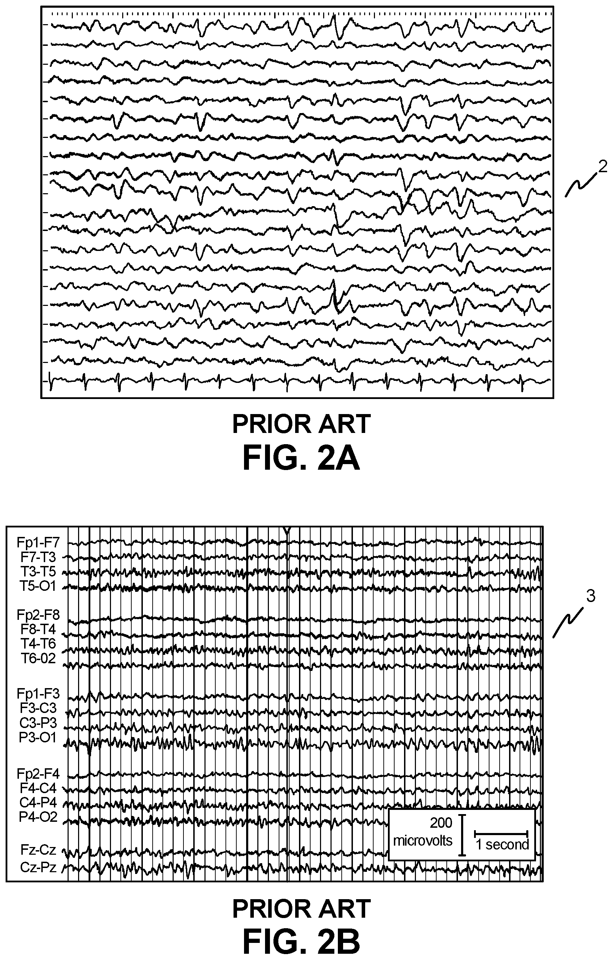

Brain waves may have various frequencies and/or bands of frequencies. "Diffuse slowing" is a hallmark indication of encephalopathy. FIGS. 2A-3D depict several EEG readings from patients experiencing symptoms of encephalopathy 2, as compared to normal controls 3. As would be apparent to one of skill in the art, in various states, brain waves in encephalopathy may be characterized by "diffuse slowing," 2 meaning that slowed waveforms can be observed on each of the channels observed. As is apparent from FIGS. 2A-2B, because this slowing s diffuse, rather than localized, the slowing (shown in FIG. 2A) is observed at most - and typically all - of the various electrodes from an EEG.

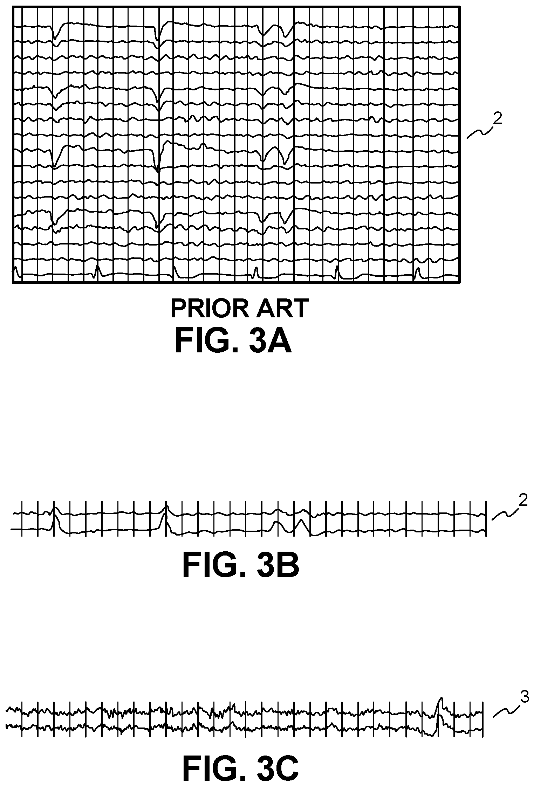

As shown in FIG. 3A, emergence of slower waves 2 as compared to the number of higher frequency waves may be an indication that a patient has or is more likely to develop encephalopathy.

As shown in FIGS. 3B-3D, because diffuse slowing can be routinely observed at all or nearly all of the EEG electrodes, it is possible to utilize fewer than the standard number of sixteen to twenty four to identify diffuse slowing and therefore predict the onset of encephalopathy. In these implementations, the two leads utilized in the BSEEG implementations discussed herein may be adequate for detecting diffuse slowing and, with appropriate signal processing and user interface, may require no special expertise for placement or interpretation, and may be performed with the aid of a simple handheld screening device.

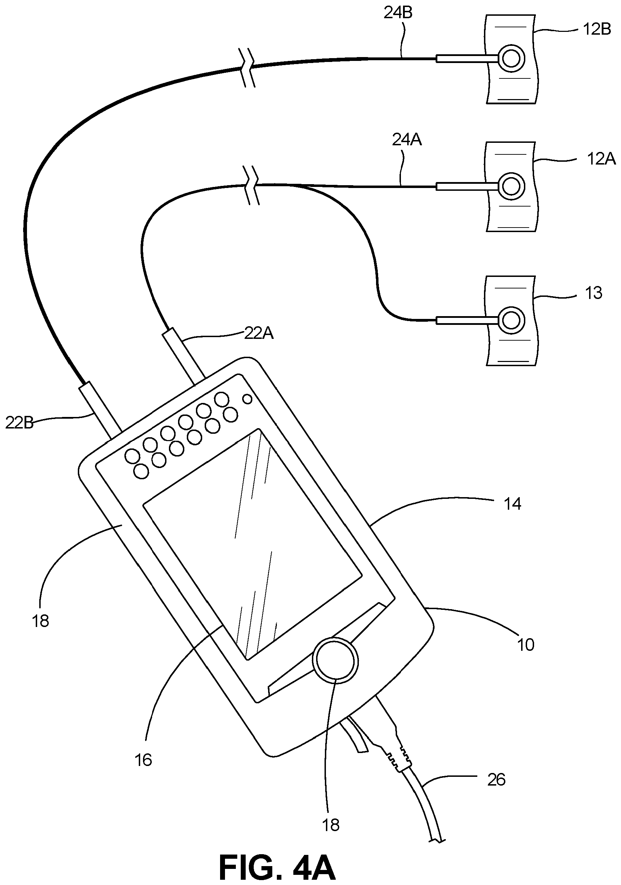

One implementation of a screening device 10 is shown in FIG. 4A. In various implementations, the systems and methods for predicting, screening, and monitoring of encephalopathy disclosed herein may utilize such a handheld or otherwise portable screening device 10. In these implementations, the screening device 10 is configured to receive signals from, for example, one or more sensors 12A, 12B. Because diffuse slowing is readily identifiable across the brain of the patient, these devices 10 are able to use far fewer than twenty sensors, such as two, three, four, five or more sensors 12. In certain implementations, between six and twenty or more sensors are used. It is therefore understood that because fewer than twenty sensors 12 are used, the disclosed devices and systems are able to be easily transported and used on a patient 30, as there is no need to apply a typical prior art EEG cap.

In the implementation of FIG. 4A, the one or more sensors 12A, 12B are brain sensors, such as, but not limited to, electrodes placed on a patient. The signals may be electroencephalograph (EEG) signals from the one or more electrodes measuring brain activity of a patient. The signals may be processed to extract one or more features of the signals. The one or more features may be analyzed to determine one or more values for each of the one or more features. The values, or a measure based on one or more of the values, may be compared against a threshold to determine the presence, absence, or likelihood of subsequent development of encephalopathy if the patient does not currently exhibit clinical signs or symptoms of the disease.

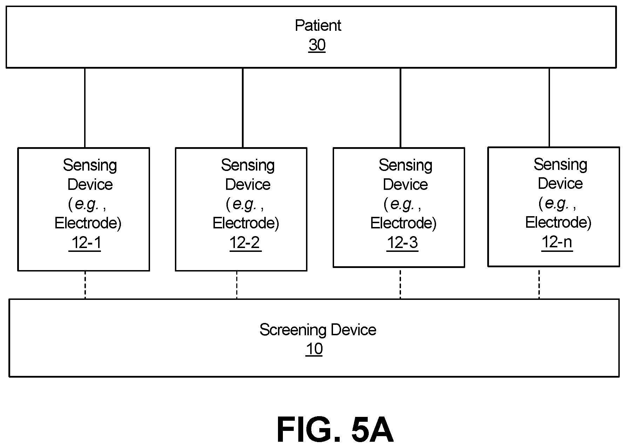

Continuing with FIGS. 4A-4B, the screening device 10 can comprise a housing 14. In various implementations, the housing 14 can be sized to be handheld or otherwise portable. The housing 14 can have a display 16 and interface 18, such as buttons or a touch screen, as would be appreciated by the skilled artisan. In various implementations, the sensors 12A, 12B are connected to the device 10 via ports 22A, 22B and wires 24A, 24B, while alternate implementations utilize a wireless interface such as a Bluetooth^{®} or the like. As shown in FIG. 4A, a ground lead 13 can also be provided, which can be bundled with one of the wires 24A, 24B to streamline application. In various implementations, a transmission cord 26 or other connection can be used to place the device 10 in electrical communication with a server or other computing device, as is discussed in relation to FIGS. 5A-B.

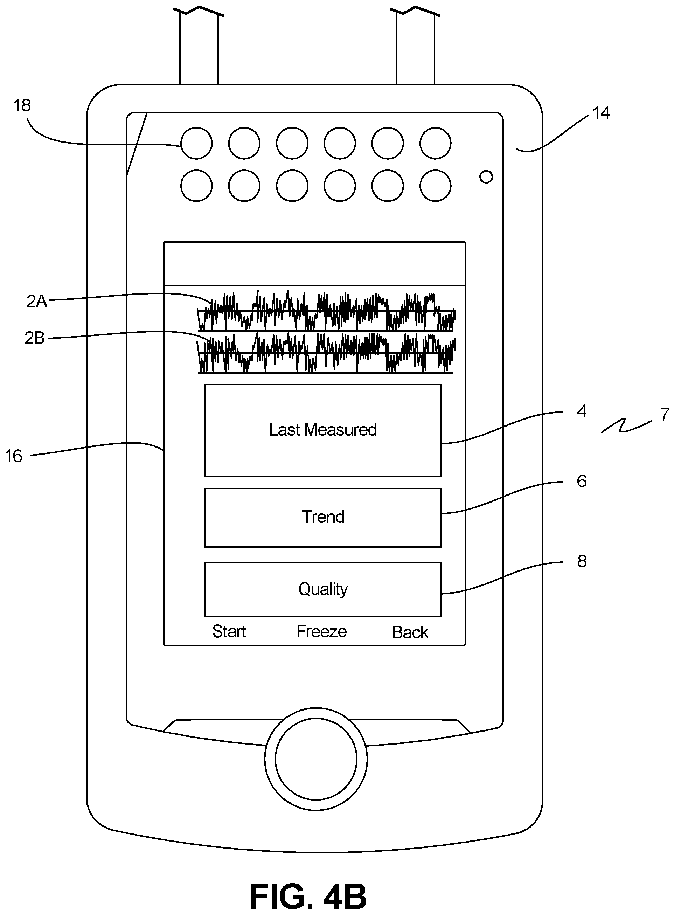

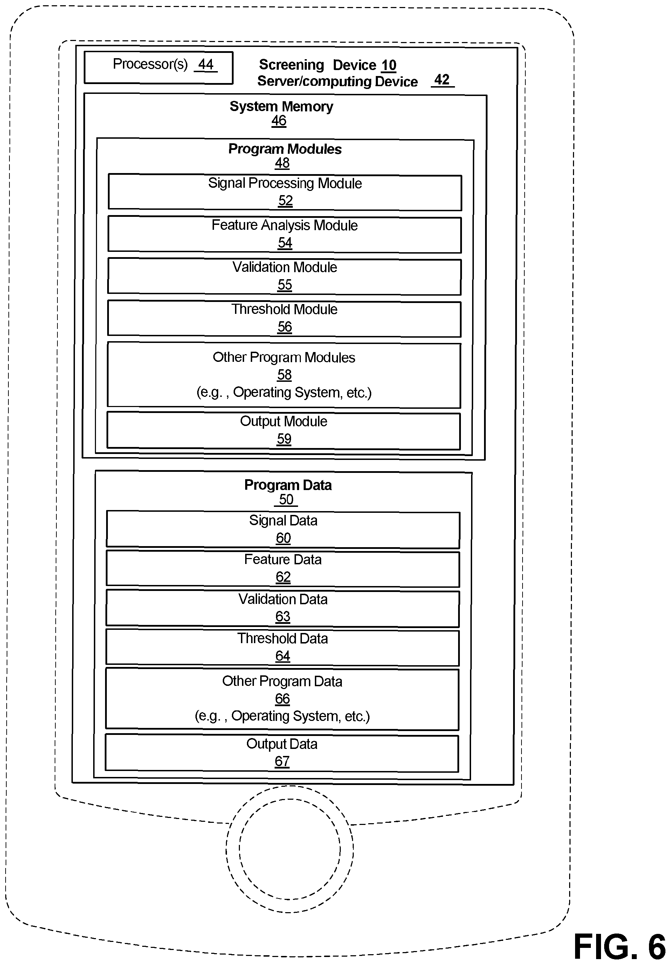

As is also shown in FIG. 4B, in various implementations the display 16 can depict graphical and / or numerical representations 7 of useful information for use in diagnosis of encephalopathy / delirium including at least one of: one or more brain waves 2A, 2B, the last measured value 4, the trend 6, signal quality 8 and the like. In exemplary implementations, a graphical representation of the threshold step described below, which compares the spectral density with an established threshold is shown as the last measured value 4. It is understood that these representations 7 can include any program data 67, and can shown to the provider as the result of understood graphical user interface techniques on the display 16. The brain waves 2A, 2B are drawn from the sensors 12 placed on the patient, and can be used to diagnose and / or predict the onset of encephalopathy in a clinical or other setting. In various implementations, the housing 14 also comprises a power supply and computing components, such as a microprocessor, memory and the like. In additional implementations, computing and other processing is done within the device housing 14, such as via program modules (described in relation to FIGS. 6-7), while additional processing can be performed elsewhere, as described herein. In each of these implementations, the screening device 10 and / or system 1 comprises a processor configured to evaluate diffuse slowing in the patient by way of spectral density analysis performed on fewer than 20 channels to identify diffuse slowing.

FIG. 5A shows an exemplary system 1 for the predicting, screening, and monitoring of encephalopathy according to one implementation. In this implementation, the system 1 may include one or more screening devices 10 (e.g., screening device 1, screening device 2, ..., screening device n), which in certain implementations is the device 10 of FIG. 1. The screening devices 10 in this implementation are operatively coupled to the one or more sensors 12A to 12-n. The one or more sensors 12 may be individual sensors, arrays of sensors, medical devices, or may be other computing devices, remote access devices, etc. The one or more sensors 12 may be one or more electrodes and/or other brain function monitoring devices. The one or more sensors 12 may be directly or indirectly coupled to a patient 30 for monitoring biological signals of the patient 30. The one or more sensors 12 may be directly coupled to and/or integral with the screening device 10 via the ports 22 (best shown in FIG. 4A).

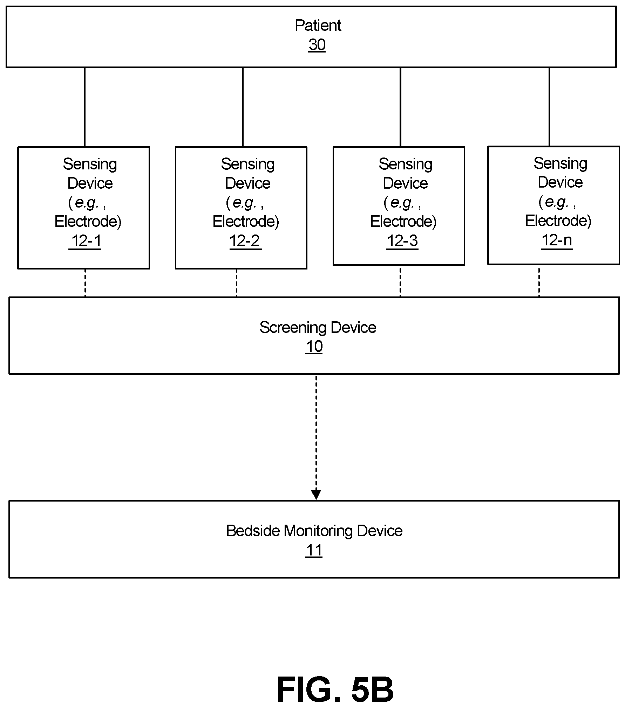

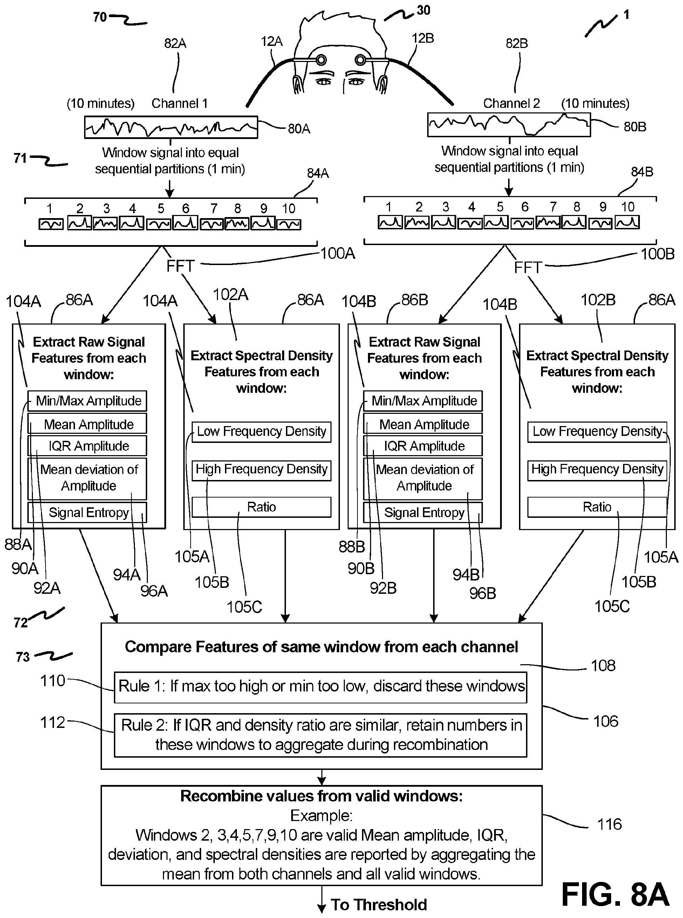

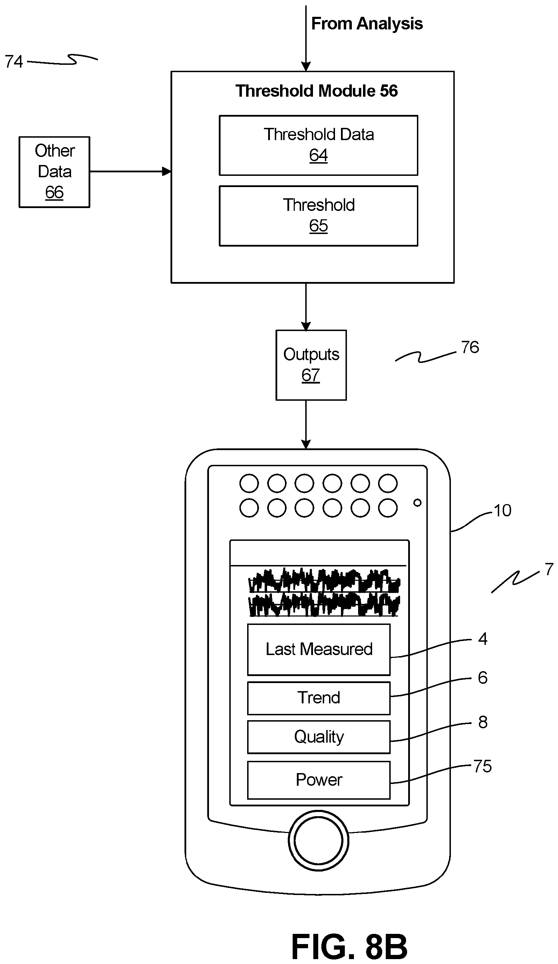

As also shown in FIG. 5A, various processing and analysis of the signals received from the sensors 12A, 12B, 12C, 12-n can be performed on the screening device 10, as are described in relation to FIGS. 6 and 8. In certain alternate implementations, such as those shown in FIGS. 5B-D, the screening device 10 can be used in conjunction with other computing devices.

As shown in the implementation of FIG. 5B, the screening device can be connected or otherwise interfaced to a bedside device 11. In various implementations, the bedside device 11 can be a patient monitor or other unified bedside monitoring device 11. In various implementations, the bedside device 11 can be used by a healthcare provider to view and / or perform certain analysis or observation steps.

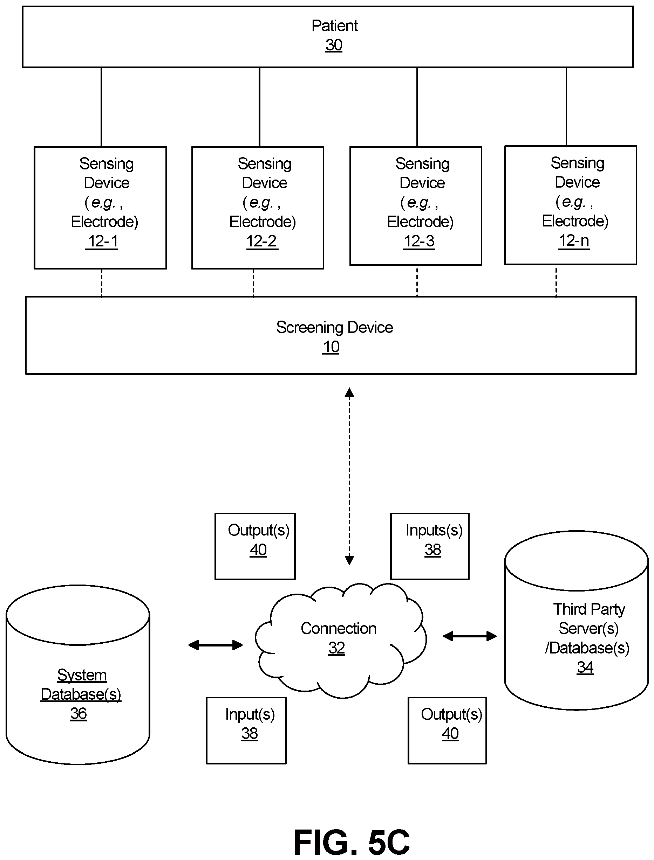

As shown in FIGS. 5C-5D, the one or more screening devices 10 may also be operatively connected directly and/or indirectly, such as over a network, to one or more servers/computing devices 42, system databases 36 (e.g., database 1, database 2, ..., database n). Various devices may be connected to the system, including, but not limited to, medical devices, medical monitoring systems, client computing devices, consumer computing devices, provider computing devices, remote access devices, etc. This system 1 may receive one or more inputs 38 and/or one or more outputs 40 from the various sensors, medical devices, computing devices, servers, databases, etc.

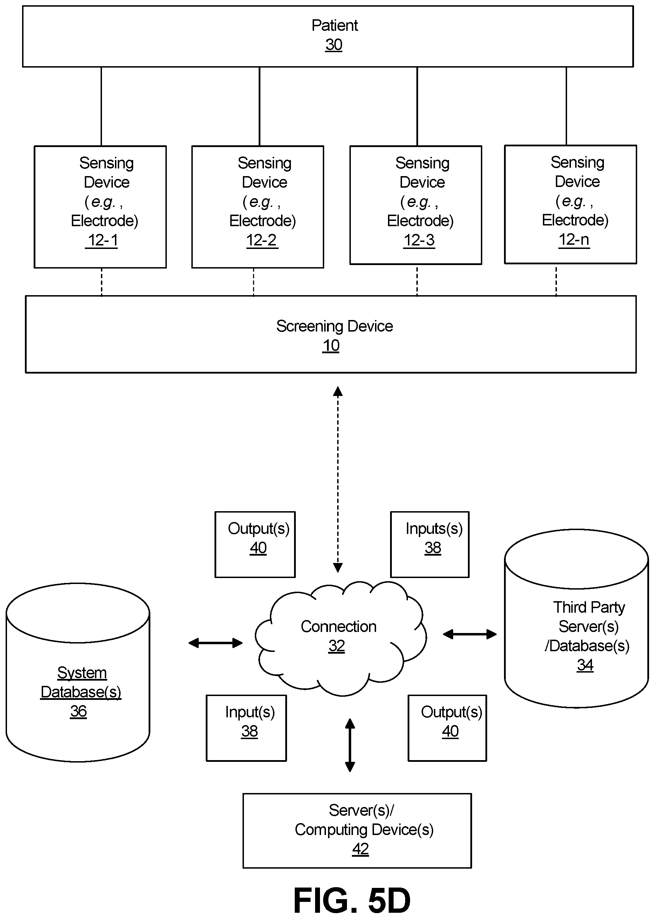

As is shown in FIG. 5D, the one or more screening devices 10 may be directly coupled to and/or integral with the one or more servers/computing devices 42 via a connection 32 and/or may be coupled to the one or more servers/computing devices 42 over one or more network connections 32. The screening device 10 and / or one or more servers/computing devices 42 may also be operatively connected directly and/or indirectly, such as over a network, to one or more third party servers/databases 34 (*e.g.,* database 1, database 2, ..., database n). The one or more servers/computing devices 42 may represent, for example, any one or more of a server, a computing device such as a server, a personal computer (PC), a laptop, a smart phone, a tablet or the like.

In various implementations, the connection 32 may represent, for example, a hardwire connection, a wireless connection, any combination of the Internet, local area network(s) such as an intranet, wide area network(s), cellular networks, Wi-Fi networks, and/or so on. The one or more sensors 12, which may themselves include at least one processor and/or memory, may represent a set of arbitrary sensors, medical devices or other computing devices executing application(s) that respectively send data inputs to the one or more screening devices 10 or servers/computing devices 42 and / or receive data outputs from the one or more screening devices 10 or servers/computing devices 42. Such servers/computing devices 42 may include, for example, one or more of desktop computers, laptops, mobile computing devices (*e.g*., tablets, smart phones, wearable devices), server computers, and/or so on. In certain implementations, the input data may include, for example, analog and/or digital signals, such as from an EEG system, other brain wave measurements, etc., for processing with the one or more servers/computing devices 10.

In various implementations, the data outputs 40 may include, for example, medical indications, recommendations, notifications, alerts, data, images, and/or so on. These may be used for collaborative projects with multiple users logging in and performing various operations on a data project from various locations. computer-based, web-based, smart phone-based, tablet-based and/or human wearable device-based.

In another exemplary implementation, the screening device 10 (and / or server/computing device 42) may include at least one processor 44 coupled to a system memory 46, as shown in FIG. 6. The system memory 46 may include computer program modules 48 and program data 50. As described above, the operations associated with respective computer-program instructions in the program modules 48 could be distributed across multiple computing devices.

Spectral density analysis from the leads can be performed by a variety of electronic and computing mechanisms. In the implementation of FIG. 6, program modules 48 are provided. These program modules may include a signal processing module 52, a feature analysis module 54, a validation module 55, a diffuse slowing or encephalopathy determination, or threshold module 56, an output module 59 and other program modules 58 such as an operating system, device drivers, etc. In various implementations, each program module 52 through 58 may include a respective set of computer-program instructions executable by processor(s) 44.

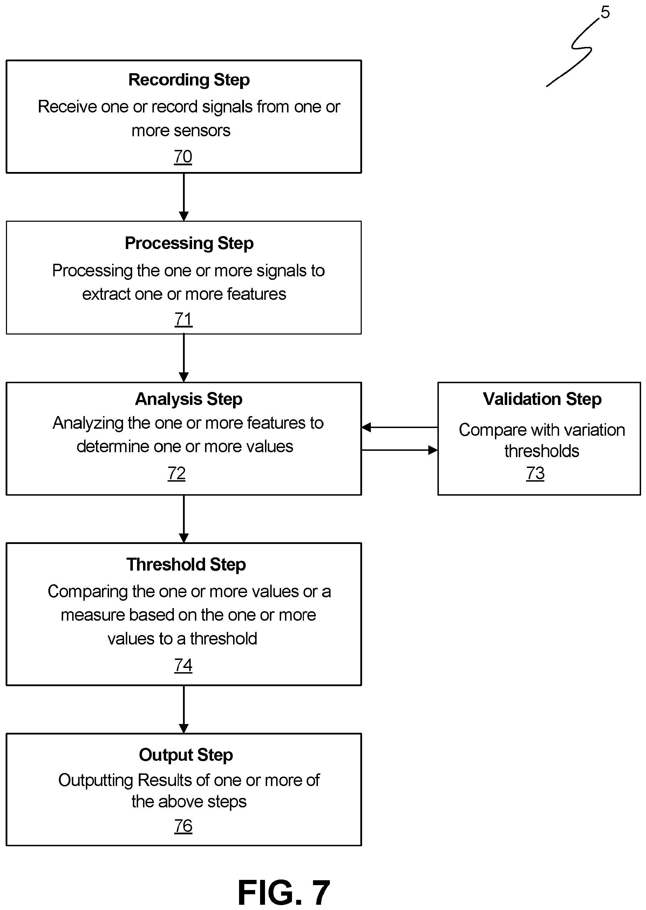

FIG. 6 depicts one example of a set of program modules and other numbers and arrangements of program modules are contemplated as a function of the particular arbitrary design and/or architecture of server/computing device 10 and/or system 1. In an exemplary implementation, program data 50 may include signal data 60, feature data 62, validation data 63, diffuse slowing or encephalopathy determination module 64, output data 67 and other program data 66 such as data input(s), third party data, and/or others configured to implement the various steps described in FIGS. 7-8.

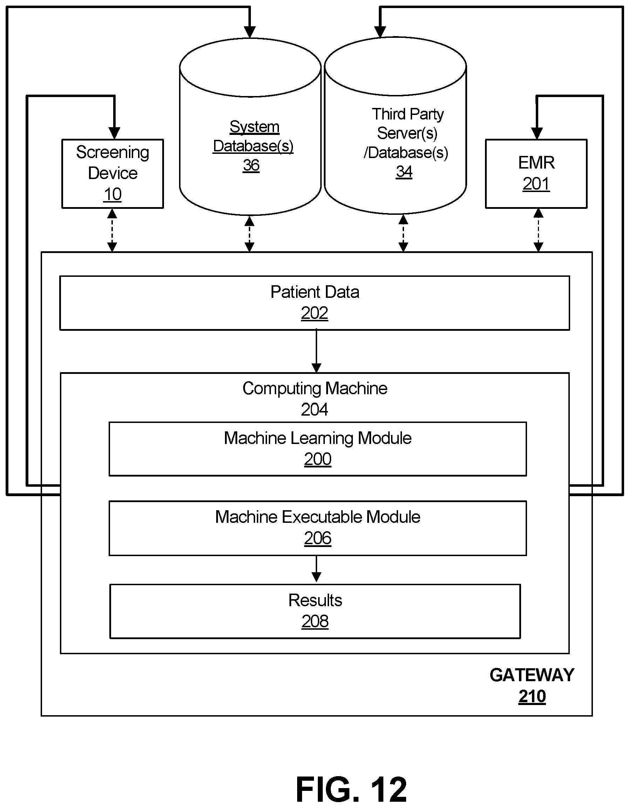

In various implementations, program data 50 may correspond to the various program modules 48 discussed above. In these various implementations, the program modules 48 and / or program data 50 can be used to record, analyze, and otherwise product output data 67 to the display (as shown at 16 in FIG. 1A and 8B) and / or any of the other computing devices described herein, such as the bedside monitoring device 11 of FIG. 5B, the various system databases 36 and / or third party servers or databases 34 of FIG. 5C, the system server(s)/computing device(s) 42 of FIG. 5D - or, in implementations where the module is not contained within the housing 14, to the screening device itself 10 - as well as the gateway 210 of FIG. 12 or any other computing, storage or communications device in electrical or physical communication with the output module. It is understood that the output data 67 provides the point of care provider with characteristics of the patient brain waves for the purposes of diagnosing or otherwise identifying the symptoms of delirium or encephalopathy. For example, in certain implementations, the provider can be provided with the last measured readings of the spectral density as well as qualitative measurements of the signal quality and trend, as has been describe elsewhere herein.

FIG. 7 is an overview of an exemplary method 5 according to an embodiment for predicting, screening, and monitoring of encephalopathy. To achieve predicting, screening, and monitoring of encephalopathy, the systems and methods may perform several optional steps.

One optional step is a recording step 70. In this step, input data, such as one or more signals may be received and / or recorded by one or all of the sensor 12, device 10, processor(s) 40 and / or system memory 46 shown, for example, in FIGS. 4A-4B, 5A-5D and 6.

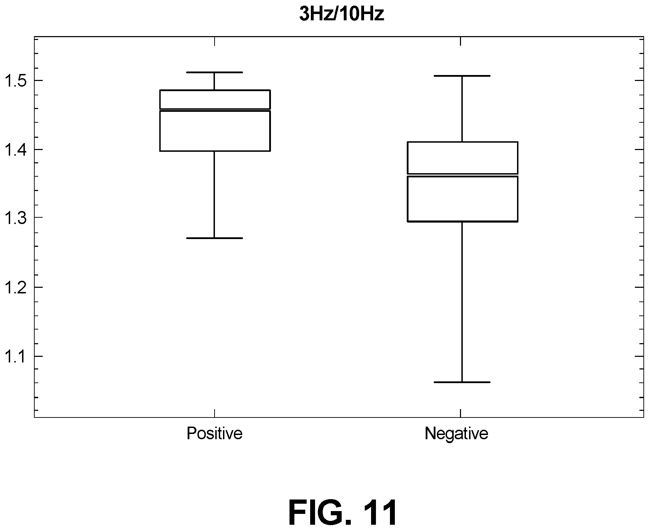

Continuing with FIG. 7, in an optional processing step 71, the one or more signals may be processed, such as by program data 50 and / or the signal processing module 52 shown in FIG. 6. In certain implementations, these signals can be processed to partition the signals into windows, as is also shown in FIG. 8A. The signals can also be processed to extract one or more values from the one or more signals and / or windows, as is described further in relation to FIG. 8A. These values can include features of the raw signals from an EEG in certain implementations. In various implementations, the system 1 is able to trend these values to improve the accuracy of the system, as is described further in relation to FIGS. 11A and 11B.

Continuing with FIG. 7, an optional analysis step 72 can be performed, where one or more values may be analyzed to determine certain features of the signals or windows. In certain implementations, this analysis step can comprise performing a Fast Fourier Transform 100 to create or otherwise compare feature data (as shown in FIG. 6 at 64).

In another optional step, a validation step 73 can be performed. In these implementations, and as shown in FIG. 8A, the spectral density 102 and / or other raw signal values 104A, 104B or other features can be used to compare the individual readings from the partitioned signal windows (shown generally at 84A, 84B) for inclusion or rejection from use in subsequent steps. In various implementations, this step can be performed by way of a validation module 55 and validation data 63. In various implementations, the validation step 73 can be performed concurrently or otherwise in repetitive iterations with the other steps described herein and utilize an error collection algorithm until the resultant signal data is ready for subsequent analysis. For example, and as described further in FIG. 8A, the validation step 73 can be used to ensure that all readings above or below certain determined predetermined error values have been excluded from further processing.

Continuing with FIG. 7, a diffuse slowing or encephalopathy determination step, or threshold step 74 can be performed. In this step, at least one of the one or more values or features or a measure based on at least one of the one or more values may be compared to an established diffuse slowing threshold (shown in FIG. 8 at 65), which can be implemented via diffuse slowing threshold data (as shown in FIG. 6 at 64). A presence, absence, or likelihood of the subsequent development of encephalopathy may be determined for a patient based on the threshold comparison. For example, and as discussed in the Examples below, using the mean of 3Hz/10Hz readings as the threshold data 64 and a threshold 65 of 1.44 (positive >=1.44 and negative <1.44), the screening device 10 may graphically indicate positive and / or negative readings, with or without the threshold 65 also being displayed. It is understood that the threshold 65 may also be modified over time as a result of system improvements and enhancements, as well as on the basis of additional data 66 and other factors.

In exemplary implementations, a graphical representation of the threshold step is displayed on the screening device 10 or other monitoring system, showing the comparison of the spectral density with the established threshold is shown as the last measured value 4. Each of these optional steps is discussed in further detail below in relation to the presently disclosed examples.

As shown in FIG. 8A, in one exemplary implementation of the system 1, a patient 30 is being monitored by sensors 12A, 12B such as by way of the screening device 10 of FIG. 1. In this implementation, the sensors 12 produce one or more signals 80A, 80B from the patient 30. The one or more signals 80A, 80B may be analog and/or digital signals. The one or more sensors 12A, 12B may be separate from the system receiving the one or more signals, or may be integral with the system receiving the one or more signals. It is understood that while the presently described system 1 relates to the collection of EEG signals, in alternate implementations the one or more sensors 12A, 12B may measure, detect, determine, and/or monitor one or more of the following physiological conditions: heart rate, pulse rate, EKG, heart variability, respiratory rate, skin temperature, motion parameters, blood pressure, oxygen level, core body temperature, heat flow off the body, galvanic skin response (GSR), electromyography (EMG), electrooculography (EOG), body fat, hydration level, activity level, oxygen consumption, glucose or blood sugar level, body position, pressure on muscles or bones, and ultraviolet (UV) radiation absorption, etc.

In various implementations, and as discussed in relation to FIGS. 4A-6, the system 1 includes one or more signal processing devices, which can be disposed within the screening device 10 or elsewhere. The processing may determine the one or more features by parsing the one or more signals to look for signal information that corresponds to certain signal features. The one or more signals may be processed to determine the presence of one or more high frequency waves and/or one or more low frequency waves. The one or more features may be types of waves, such as, for example, but not limited to, high frequency waves and/or low frequency waves.

In the implementation of FIG. 8A, the signals 80A, 80B are received through first 82A and second 82B channels for processing, as described above. Other implementations are possible. The methods may be integrated into 8-bit or 16-bit embedded device environments, or more robust environments as well. The methods may be integrated into existing hospital patient workflows.

As also shown in FIG. 8A, in certain implementations the signals 80A, 80B can be processed to window the signal into equal sequential partitions (shown generally at 84A and 84B). In the depicted implementation, a signal duration of ten minutes is shown, wherein the signal is windowed into ten individual one minute windows (also shown generally at 84A and 84B), however it is understood that in alternative implementations other signal durations and numbers of windows can be used. In various implementations, the signals may be less than a second long or longer than an hour, or any number of minutes. Similarly, there can be any number of windows, and the windows can range in time from fractions of a second to many minutes or more.

In the implementation of FIG. 8A, after the signals 80A, 80B have been partitioned, several optional processing and analysis steps can be performed, as described in relation to FIG. 7. In various implementations, the values 86A, 86B are extracted, and various optional steps can be performed to values 86A, 86B to identify raw data features 104A, 104B such that some or all of the minimum / maximum amplitude 88A, 88B, the mean amplitude 90A, 90B, the interquartile range (IQR) amplitude 92A, 92B, the mean deviation of amplitude 94A, 94B and / or signal entropy 96A, 96B can be established for the respective signal 80A, 80B. As would be apparent to one of skill in the art, other raw signal features 104A, 104B can be identified.

In an analysis step, spectral density analysis 102 can be used to identify diffuse slowing. As is also shown in FIG. 8A, a Fast Fourier Transform 100 may be performed on the one or more signals 80A, 80B to identify additional features 104A, 104B. The Fast Fourier Transform 100 may be used alone or in combination with other analysis tools to assess spectral density. A spectral density analysis 102 may be performed on the one or more signals to determine one or more spectral density features 104A, 104B, such as low frequency density 105A, high frequency density 105B and the ratio 105C of the low and high frequency densities. These features 104A, 104B from spectral density analysis 102 may be used alone or in conjunction with the various values 104A, 104B drawn from the raw signal to predict and / or diagnose encephalopathy by using a variety of optional steps and combinations.

In certain implementations, a spectral density analysis 102A, 102B can be performed on each of the one or more signals 80A, 80B, such as the EEG signals depicted, to differentiate different patient states. Spectral density analysis 102A, 102B may provide values 104A, 104B including the ratio 105C of high frequency brain electrical activity to low frequency brain electrical activity. For example, the ratio of about 10Hz signals to signals of about 2Hz, 3Hz, or 4Hz can be compared to establish diffuse slowing. One or more bands or windows within the one or more signals 80A, 80B may be identified for use in the systems and methods described herein.

In certain implementations, a validation step 73 can be performed (shown in box 106). In these implementations, the spectral density 102 and / or other raw signal values 104A, 104B can be used to compare the individual readings from the partitioned signal windows (shown generally at 84A, 84B) for inclusion or rejection from the analysis. For example, in certain implementations a correction algorithm 108 can be performed. In one implementation, the error collection algorithm 108 performs a number of optional steps. In one optional step, various values 104A, 104B above or below certain pre-determined error thresholds within a given window are discarded 110. In another optional step, if the IQR and / or density ratio 105C are within a certain pre-determined proximity 112, these window signals are retained for aggregation and recombination 116, as described below. Other optional steps are possible.

In these implementations, an optional additional recombination step 116 can be performed. In the recombination step 116, windows 84A, 84B that have not been removed as a result of the validation step 73 (box 106) can be combined, such that the values 104A, 104B, 104A, 104B from those windows are aggregated as diffuse slowing threshold data 64.

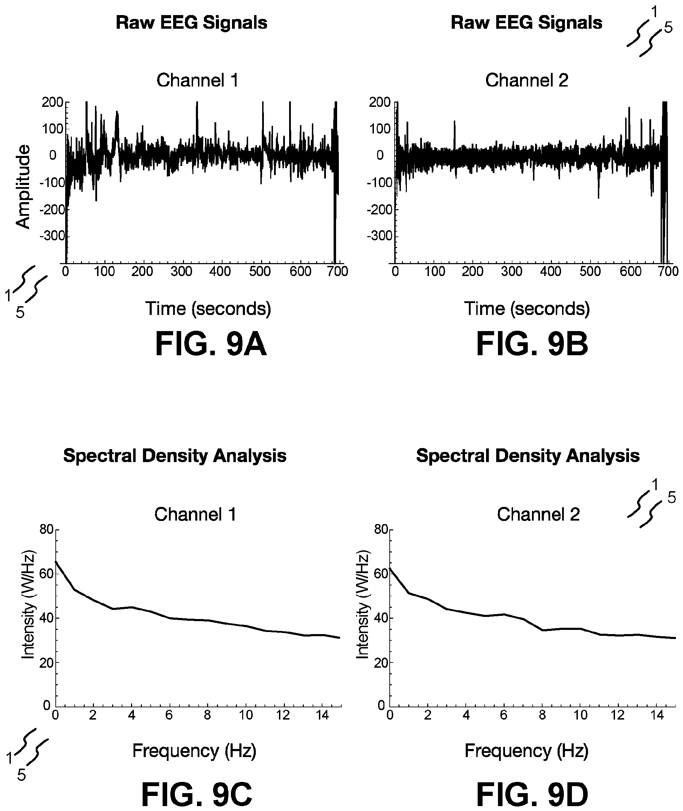

Using the diffuse slowing threshold data (shown in FIG. 6 at 64), a diffuse slowing or encephalopathy thresholding step 74 is performed, as is further demonstrated in FIGS. 9A-13. In these implementations, the aggregated threshold data 64 can be compared against an established threshold 65 by way of the threshold module 56. In various implementations, data from other sources 66, such as electronic medical records, can also be compared against thresholds 65 to establish outputs 67, which can be graphically presented, for example, on any of the other devices and systems described herein, such as in relation to FIGS 5A-5D. One such example is the display of the output data 67, which can include the last measured value 4, trend 6, quality 8, power 75 and other graphical representations, such as those found in FIGS. 9A-D.

As was shown in FIGS. 2A-3D, brain waves in encephalopathy may be characterized by "diffuse slowing", meaning that electrodes, preferably all electrodes, from an EEG show slowed waveforms. As noted above, brain waves may have various frequencies and/or bands of frequencies. Diffuse slowing may mean that slower waves, those with lower frequency, are seen across most, if not all, electrodes on an EEG. Emergence of slower waves as compared to the number of higher frequency waves may be an indication that a patient has or is more likely to develop encephalopathy. Two leads (and a ground) may be adequate for detecting diffuse slowing and, with appropriate signal processing and user interface, may require no special expertise for placement or interpretation.

As shown in FIGS. 9A-9B, spectral density analysis 102A, 102B is performed on one or more values 104A, 104B that may be the number of high frequency waves in the one or more signals and/or the number of low frequency waves in the one or more signals. As shown in FIG. 9A, each channel 82A, 82B can be analyzed by the steps described in relation to FIG. 8A to determine the frequency of "high" and "low" frequency waves and other characteristics as output data 67. In various implementations, these representations can be used as any of the various forms of data discussed above in the process, and can be depicted on the device display 16 (shown in FIG. 4B).

The values 104A, 104B may be computed over the entirety of the one or more signals 80A, 80B as part of any of the steps described above. The values 104A, 104B may be computed over a subset of the one or more signals or a subset of time of the one or more signals. For example, if the one or more signals are five minutes in duration, the values 104A, 104B may be computed over less than five minutes, such as four minutes, three minutes, two minutes, one minute, thirty seconds, etc. Therefore, the one or more values 104A, 104B may be a feature 104A, 104B and / or values 104A, 104B over a predetermined amount of time. The one or more values 104A, 104B may be a number of high frequency waves over a period of time and/or a number of low frequency waves over a period of time. The one or more values 104A, 104B may be a ratio of a number of high frequency waves to a number of low frequency waves. The one or more values 104A, 104B may be a ratio of a number of high frequency waves over a period of time to a number of low frequency waves over a period of time.

As shown in FIGS. 8A-B and 9A-D, in various implementations of the device 10, system 1 and method 5, the ratios of high and low frequency waves for each channel can be plotted and compared at the diffuse slowing or encephalopathy determination step (shown in FIG. 7 at 74). In these implementations, at least one of the one or more values or features or a measure based on at least one of the one or more values contained in the threshold data 64 may be compared to an established diffuse slowing threshold 65 via the module 56. A presence, absence, or likelihood of the subsequent development of encephalopathy may be determined for a patient based on the comparison. In various implementations, the frequency is compared over time for each signal, and the results analyzed. As described further below, FIGS. 9A and 9B show the raw EEG channel signals over time for each of the respective channels. FIGS. 9C and 9D depict the resulting spectral density analysis 102 for each channel.

Experimental results are demonstrated in the accompanying examples and conclusions are given.

### Example 1: Encephalopathy Screening via BSEEG Compared to Clinical Diagnosis of Delirium

In this Example, a preliminary study was performed, utilizing more than 80 patients aged 65 and older - both with and without clinical a diagnosis of delirium - to compare their brain wave signals obtained by the screening device 10, system 1 and method 5. Baseline cognitive function was assessed using the Montreal Cognitive Assessment (MoCA).

In this Example, patients were then screened for the presence of delirium with Confusion Assessment Method for the Intensive Care Unit (CAM-ICU). Following evaluation, EEG readings were taken using the presently described devices, systems and method by BSEEG, that is, placing two EEG leads on patients' foreheads - one per hemisphere - to obtain two-channel signals from the right and the left over the course of 10 minutes. A ground lead was also used. This process was repeated twice a day during their hospitalization, up to 7 days, and testing was terminated if no change in mental status is observed after that time. Where mental status changes were observed, changes were monitored for additional time.

In this Example, the quality of the EEG signal from the presently described screening device was compared with the EEG signal obtained from a traditional 20-lead EEG machine for the same patients at the same time. It was established that there was no significant difference between the results.

In this Example, a further preliminary analyses of the data from a limited number of cases involving patients with and without delirium. Initial analysis showed that the presently described devices, systems and methods clearly differentiated these patients, and also detected delirium and a lack of delirium in the same patient at different times. Based on the BSEEG results and signal-processing algorithm, we count the number of subjects correctly classified as positive (true positive) and as negative (true negative). In this Example, by tabulating the number of cases incorrectly categorized as positive (false-positive) and as negative (false-negative). It is possible to calculate sensitivity and specificity with several thresholds for positive and negative results as compared with the CAM-ICU findings.

Receiver Operating Characteristic (ROC) analysis was subsequently conducted and an algorithm was developed to evaluate the screening device output data. The ROC process was repeated with multiple algorithms to develop the best algorithm with a target Prediction Accuracy (AUC) of more than 0.7 (with 1.0 being perfect). In various implementations, the algorithm can be implemented into the system, such as in the handheld screening device 10 (shown for example in FIG. 4A) or elsewhere in the system 5 for use in bedside diagnostics and system improvement, as described above.

### Example 2: Screening Device Assessment

In this Example, the initial training set of dataset contained 186 total patient EEG samples correlated with clinical or CAM evidence of delirium. These samples represented 5 positive, 179 negative and two negative cases in which the data quality was inadequate for analysis to be performed were therefore excluded from further review.

In this Example, a 15Hz low-pass filter was originally used, but the preliminary results indicated unequal dampening in the FFT frequency information between the positive and negative cases, therefore the low-pass filter eliminated.

During processing of the processed samples, it was observed that windows of 4 seconds were sufficient to demonstrate good results. Also, in this Example, windows containing high amplitude peaks were excluded using threshold of 500µV for example as shown in FIGS. 9A and 9B.

FIGS. 9C and 9D depict the spectral density for the channels, wherein the intensity (in W/Hz) can be compared to each frequency (in Hz) to establish the low:high frequency ratio. It was observed that using a ratio of 3Hz/10 Hz yielded preferable predictive results in this training set as opposed to either 2Hz/10Hz or 4Hz/10Hz ratios.

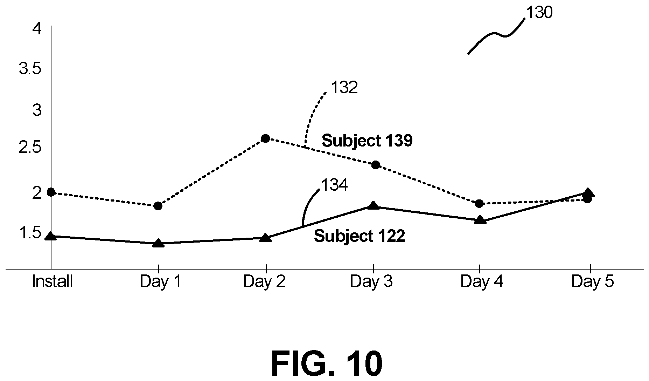

In FIG. 10, the system 1 and method 5 can be used to establish a time series 130 spectral density analysis to compare delirious 132 to "normal" 134 wave frequencies over time. As is apparent from delirious Subject 139, the system is able to identify an increase in the ratio of low (3Hz) to high (10Hz) wave forms as compared with the control (Subject 122), thereby indicating the presence of encephalopathy. In various implementations, these results can be used to prospectively identify the onset of encephalopathy by establishing the spectral density as compared to population thresholds.

As shown in FIG. 11, using the mean of 3Hz/10Hz as the threshold data 64 with a threshold 65 set at 1.44 (positive >=1.44 and negative <1.44), the performance metrics as opposed to the CAM-ICU (or otherwise clinically identified patients) are shown in Table 1:

**Table 1: Diffuse Slowing Assessment Compared With Clinical Diagnosis**

| | |
|---|---|
| True Positives | 4 |
| False Positives | 22 |
| True Negatives | 157 |
| False Negatives | 1 |
| Accuracy | 87.50% |
| Sensitivity | 80.00% |
| Specificity | 87.70% |
| Positive Predictive Value | 0.153846 |
| Negative Predictive Value | 0.993671 |

It is understood that numerous additional examples can be provided.

### Example 3: Machine Learning

As shown in FIG. 12, in certain implementations a machine learning model (box 200) is used to identify characteristics of delirium / encephalopathy, and can be used to revise the other systems, methods and devices described herein, such as by refining the threshold (described in relation to FIGS. 6-8 at 65) to improve the accuracy of the diagnosis. In these implementations, a model is used to associate personal and population patient data (shown generally at box 202) within a computing machine (box 204). Generally, the various machine learning approaches, may be coded for execution on the screening device 10, server/computing device 42, a database 36 third party server 34 other computing or electronic storage device in operable communication with the screening device 10 and / or sensors 12 (also shown in the implementations of FIGS. 5A-5D).

The model may be executed on data (box 202) recorded or otherwise observed from patients 30 (such as the spectral density analysis 102, output data 67 and other values described in relation to FIG. 8A and the like) as well as from other recorded data, such as from electronic medical records (EMR - box 201).

In various implementations, the EMR data 201 may include, but is not limited to, one or more of the following physiological conditions: heart rate, pulse rate, EKG, heart variability, respiratory rate, skin temperature, motion parameters, blood pressure, oxygen level, core body temperature, heat flow off the body, GSR, EMG, EEG, EOG, body fat, hydration level, activity level, oxygen consumption, glucose or blood sugar level, body position, pressure on muscles or bones, and UV radiation absorption, etc. The out observed data output from these combined EMR and other systems described herein may also be provided to an EMR, a separate patient monitoring system, a graphical user interface on the device(s), etc.

Accordingly, the various systems and methods using the machine learning model (box 200) may send and / or receive information from various computing devices, as well as a patient's EMR for use in monitoring, screening, or predicting of delirium by way of a gateway 210 or other connection mechanism. The systems and methods may utilize EMR data to improve accuracy of the monitoring, screening, or predicting of delirium performed in conjunction with the screening device 10 and associated systems 1 and methods 5.

In various implementations, patient data 202 may also be loaded on to any of the computer storage devices of a computer to generate an appropriate tree algorithm or logistic regression formula. Once generated, the tree algorithm, which may take the form of a large set of if-then conditions, may then be coded using any general computing language for test implementation. For example, the if-then conditions can be captured and compiled to produce an machine-executable module (box 206), which, when run, accepts new patient data 202 and outputs results 208, which can include a calculated prediction or other graphical representation (box 208). The output may be in the form of a graph indicating the prediction or probability value along with related statistical indicators such as p-values, chi-scores and the like. In various implementations, these results 208 can be re-introduced into the learning module 200 or elsewhere to continually improve the functions of the system, including by updating the various thresholds used throughout. It is understood that these implementations are also able to trend the respective data values and readings to improve the performance of the device, system and methods. In these implementations, for example, a continuous stream of trend data that can be used to provide additional optional evaluation steps, and trends over time can be identified. In various implementations, the model can provide additional program data (shown in FIG. 6 at 48) to improve accuracy, as well as be included in aggregation, shown in FIG. 8 at 116.

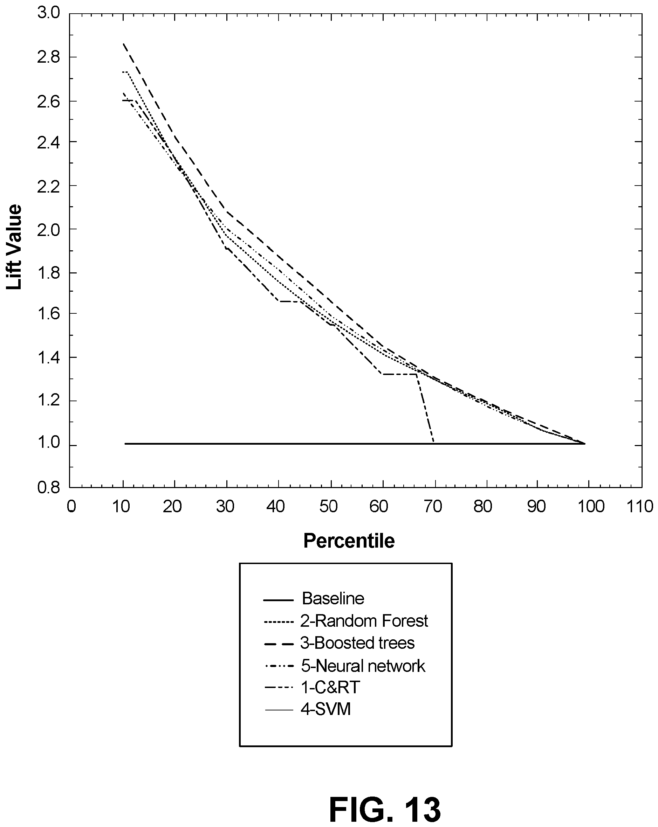

To produce better algorithms and to further determine the importance of variables in the machine learning model (box 200), enhanced classification and regression tree approaches may be used. For example, classification & regression trees, random forest, boosted trees, support vector machines, neural networks may be used, as well as other machine learning techniques previously described. A lift chart showing the lift value of each of these approaches is shown in FIG. 13.

A tree boosting approach combines a set of classifier variables to achieve a final classifier. In various implementations, this is done by constructing an initial decision tree based on the model development data to classify the dependent variable. For all cases in the development data set in which the outcome is mis-classified, the weight of these cases is increased (boosted), and a new decision tree is generated to optimize classification of the outcome based on the new case weights. The mis-classified cases again have their weights boosted, and a new decision tree is generated. This approach is repeated iteratively, typically hundreds or thousands of times, until an optimal boosted tree is identified. This boosted decision tree is then applied to the validation data set, and cases in the validation data set are classified as responders or non-responders. Many other known approaches can be used, such as

It should be noted that the boosted tree machine learning approach as well as any of the more sophisticated tree generating approaches, may produce very complex algorithms (containing many if-then conditions), as has been previously described. Instead, the selection of variables used as inputs into any of the regression and classification tree techniques to generate an algorithm and/or the relative importance of the variables also uniquely identify the algorithm.

In this Example, a machine learning algorithm (like that of box 200) was developed on a cohort of 13,819 patients. In this example, variables such as laboratory values, medications, age were utilized. The algorithm was trained on 12,461 of these patients and validated on 1,358 test cases. The delirium outcome in each patient was compared to the DOSS scale, a manually administered screening tool where a score of greater than 3 indicates delirium.

In this Example, model performance was as follows: true negatives: 951; false positives: 62; true positives: 132; false negatives: 213. Accordingly, the observed error rate was 20%, the accuracy was 80%. Importantly, this model did not include output from the screening device 10. It is understood that when used in conjunction with the screening device 10, the accuracy can be increased above 80%. Various additional implementations are possible, as are shown in FIG. 13.

*Values, features and threshold.* As used herein, the terms "value" and "features" can be interchangeable, and contemplate raw and analyzed data, be it numerical, time-scale, graphical, or other. In various implementations like those described herein, the value, such as the number of high frequency waves may be compared against a threshold. Alternatively, or in addition, a ratio of two or more values may be compared against a threshold. The threshold may be a predetermined value. The threshold may be based on statistical information regarding the presence, absence, or likelihood of subsequent development of delirium, such as information from a population of individuals. The threshold may be predetermined for one or more patients. The threshold may be consistent for all patients. The threshold may be specific to one or more characteristics of the patient, such as current health, age, gender, race, medical history, other medical conditions, and the like. The threshold may be adjusted based on physiological data in a patient's electronic medical record (EMR).

The threshold may be a ratio of high frequency waves to low frequency waves. The threshold may be a ratio of high frequency waves over a period of time to low frequency waves over a period of time. Throughout the disclosure herein, the ratio is referred to as the ratio of high frequency waves to low frequency waves, but it is understood that the ratio could also be the ratio of low frequency waves to high frequency waves as long as the format of the ratio is consistent throughout out the process. For example, the comparison may be between a ratio of high frequency waves to low frequency waves or the reverse, *i.e.,* low frequency waves / high frequency waves.

The one or more features or values may be predetermined. For example, the range for waves that are high frequency waves may be predetermined as being greater than a set value. Similarly, the range for waves that are low frequency waves may be predetermined as being less than a set value. The set values may be the same for all patients or may vary depending on specific patient characteristics.

Other features or values of the one or more signals may be extracted. For example, signal to noise ratios may also be determined for other uses. Data quality may be assessed by looking for non-physiologic frequencies of electrical activity. Data collection and/or interpretation may be limited to stopped when data quality is below an acceptable level.

*Device characteristics and signal collection.* The systems and methods described herein may provide a special-purpose screening device 10, system 1 and method 5 that is/are simple, convenient, and easy to use. The systems and methods may utilize electroencephalogram (EEG) technology that is simplified for an end user. The systems and methods may automatically interpret data and provide guidance to a medical professional regarding the monitoring, screening, or subsequent development of delirium by a patient. Traditionally, EEG data is visually inspected by a trained neurologist and no automation of the process is performed. Options may exist for interfacing with standard monitoring equipment, mobile devices, cloud technologies, and others to create an automated process.

The methods and systems describe herein may be useful in various medical areas, such as, but not limited to, intensive care, pre- and post-surgical care, geriatrics, nursing homes, emergency room and trauma care. Monitoring, screening, or predicting may improve patient care while in a hospital or other healthcare setting. Patients may also utilize personal health care devices and monitoring to allow for monitoring of their condition remotely when not in a healthcare setting. For example, personal healthcare devices may monitor patients at home or other locations outside a healthcare setting and provide monitoring, screening, or predicting of delirium. Remote sensing and/or analysis systems may interface with systems utilized by healthcare professionals.

As shown in the various implementations, the one or more sensors 12 may be placed in communication with a patient 30. The one or more sensors 12 may be one or more brain sensors, such as, but not limited to, EEG devices, such as one or more EEG leads / electrodes. For purposes of this disclosure the terms "leads" and "electrodes" are used interchangeably. The one or more signals may be EEG signals. EEG signals may include voltage fluctuations resulting from ionic current within neurons of the patient's brain. There may be a plurality of sensors. There may be two sensors, such as two EEG electrodes. The use of less than the traditional 16 or 24 electrode EEG systems may provide for reduced costs and complexities for predicting, screening, or monitoring of delirium. In various implementations, 2 or more leads or sensors are used. In certain implementations, 2, 3, 4, 5, 6, 7, 8, 9 or 10 sensors are used. In additional implementations, 11, 12, 13, 14 or 15 sensors are used. In yet further implementations, more than 15 sensors are used. In various implementations, a minimal number of easily placed EEG leads may be used - less than is demonstrated in the prior art - thereby eliminating and/or reducing the need for a skilled EEG technician and/or a sub-specialized neurologist. In various implementations, at least 1 ground lead is used, and in alternate implementations more than 1 ground lead is used, such as 2, 3, 4, 5, 6, 7, 8, 9, 10, 11, 12, 13, 14, 15, 16 or more ground leads.

The one or more sensors 12 may be non-invasive. Non-invasive electrodes may be placed on the skin of a patient. There may be two skin contact points at a minimum, *i.e.,* the sensor and the grounding sensor. There may be passive sensors in that there will be no external electrical current running through these sensors. Electrically active sensors may be used. The one or more sensors may be adhesive patches with printed circuitry or a non-adhesive headset that couples the sensors to the skin. The one or more sensors may be placed on the head of a patient, such as on a forehead and/or behind one or more of the patient's ears. A minimum separation between the one or more sensors may be provided, such that the one or more sensors are not in contact with each other.

Minimally invasive or invasive sensors may be used. The minimally invasive or invasive sensors may provide the one or more signals as an indication of a physiological condition, such as brain activity.

The one or more signals may be converted from analog signals to digital signals, if necessary. The conversion may be performed prior to the one or more signals being received by the processing device, at the processing device, or at a separate device. If the one or more signals are made or received as digital signals, no conversion may be necessary.

The one or more signals may be indicative of one or more brain functions of a patient. The one or more signals may provide information regarding brain wave activity of the patient. Brain waves may be measured for a patient. rain waves may be performed by EEG, which may be a recording of the electrical activity of the brain from the scalp. The recorded waveforms may reflect cortical electrical activity. Signal intensity for EEG may be small, and may be measured in microvolts. Traditionally, there are several frequencies and/or bands of frequencies that may be detected using EEG. The definition of high frequency and low frequency may vary depending on various factors including, but not limited to the patient population. The definition of high frequency and low frequency may be consistent across patient populations. Low frequency waves may be waves with less than approximately 7.5 Hz, less than, approximately 7.0 Hz, less than approximately 6.5 Hz, less than approximately 5.5 Hz, less than approximately 5 Hz, less than approximately 4.5 Hz, less than approximately 4.0 Hz, less than approximately 3.5 Hz, or less than approximately 3.0 Hz. High frequency waves may be waves with more than approximately 7.5 Hz, more than approximately 8.0 Hz, more than approximately 8.5 Hz, more than approximately 9.0 Hz, more than approximately 9.5 Hz, more than approximately 10.0 Hz, more than approximately 10.5 Hz, more than approximately 11.0 Hz, more than approximately 11.5 Hz, more than approximately 12.0 Hz, more than approximately 12.5 Hz, more than approximately 13.0 Hz, or more than approximately 14.0 Hz.

The one or more signals may be real-time or near real-time streams of data. The one or more signals may be measured and/or stored for a period of time before processing and/or analysis.

Although not required, the systems and methods are described in the general context of software and/or computer program instructions executed by one or more computing devices that can take the form of traditional servers/desktops/laptops; mobile devices, such as a Smartphone or tablet; wearable devices, medical devices, other healthcare systems, etc. Computing devices may include one or more processors coupled to data storage for computer program modules and data. Key technologies may include, but are not limited to, multi-industry standards of Microsoft and Linux/Unix based Operating Systems; databases such as SQL Server, Oracle, NOSQL, and DB2; Business Analytic/Intelligence tools such as SPSS, Cognos, SAS, etc.; development tools such as Java,.NET Framework (VB.NET, ASP.NET, AJAX.NET, etc.); and other e-Commerce products, computer languages, and development tools. Such program modules may include computer program instructions such as routines, programs, objects, components, etc., for execution by the one or more processors to perform particular tasks, utilize data, data structures, and/or implement particular abstract data types. While the systems, methods, and apparatus are described in the foregoing context, acts and operations described hereinafter may also be implemented in hardware.

*Analysis and diagnosis.* In certain embodiments, the presence of delirium may be confirmed by the various devices, systems and methods described herein. The absence of delirium may be confirmed or determined by the systems and methods described herein.

A patient 30 may currently be diagnosed as having delirium. Healthcare professionals may want to monitor the status of the patient's delirium. The method and system may provide an efficient and cost effective system and method for monitoring the status of the patient's delirium. The method and system may allow for healthcare professionals to determine whether a patient's delirium is improving, remaining stable, or worsening. A healthcare professional may not be certain whether a patient has delirium. For example, a patient may have some, but not all, of the clinical signs and symptoms associated with delirium.

A patient 30 may not currently be diagnosed with delirium, and may not currently possess one or more of the clinical signs and symptoms of delirium. For purposes of this disclosure, "clinical signs and symptoms of delirium" may be defined according to the DSM-5 Criteria for Delirium (American Psychiatric Association (2013). Diagnostic and Statistical Manual of Mental Disorders (5^{th} ed.). Washington, DC). In particular, the clinical signs and symptoms of delirium may be as follows:
A. A disturbance of attention (*i.e.,* reduced ability to direct, focus, sustain, and shift attention) and awareness (reduced orientation to the environment).
B. The disturbance develops over a short period of time (usually hours to a few days), represents a change from baseline attention and awareness, and tends to fluctuate in severity during the course of a day.
C. An additional disturbance in cognition (*e.g*., memory deficit, disorientation, language, visuospatial ability, or perception).
D. The disturbances in Criteria A and C are not better explained by a pre-existing, established or evolving neurocognitive disorder and do not occur in the context of a severely reduced level of arousal, such as a coma.
E. There is evidence from the history, physical examination or laboratory findings that the disturbance is a direct physiological consequences of another medical condition, substance intoxication or withdrawal, or exposure to a toxin, or is due to multiple etiologies.

A patient 30 may not be considered to be diagnosed with delirium unless all of criteria A-E are met. Less than all of criteria A-E must be met before a patient is considered to be diagnosed with delirium. A patient may be considered to be diagnosed with delirium if criteria A-C are met. A patient may be considered to be diagnosed with delirium if two or more of criteria A-E are met. A patient may be considered to be diagnosed with delirium if three or more of criteria A-E are met. A patient may be considered to be diagnosed with delirium if they meet criteria A or criteria C. A patient may be considered to be diagnosed with delirium if they meet criteria A or criteria C and at least one of criteria B, D or E. A patient may be considered to not be diagnosed with delirium if three or less of criteria A-E are met.

For purposes of the present disclosure, a patient 30 may not have clinical signs and symptoms of delirium if they have not currently been diagnosed as having delirium by a medical professional. The conditions noted above are the current guidelines for diagnosis of delirium based on the DSM-5. New and/or updated versions of these guidelines or other guidelines may be used to determine whether a patient has delirium, and may have different clinical signs and symptoms. The devices 10, systems 1 and methods 5 herein may predict a patient developing delirium prior to showing one or all clinical signs and symptoms regardless of the criteria utilized for the diagnosis.

There may be provide systems and methods for predicting subsequent development of delirium by the patient when clinical signs and symptoms of delirium may not currently exist in the patient.

A prediction may be made regarding a likelihood of subsequent development of delirium for the patient when the patient is not currently diagnosed with delirium. The patient may not currently have one or more of the clinical signs and symptoms of delirium at the time of determining the likelihood of subsequent development of delirium.

An indication of the presence, absence, or likelihood of the subsequent development of delirium may be output for the patient. The output may take various forms, including a notification, an alert, a visual indication, an auditory indication, a tactile indication, a report, an entry in a medical record, an email, a text message, and combinations thereof.

The indication of the presence, absence, or likelihood of the subsequent development of delirium may be a binary indication, such as, for example, a "yes" or "no" indication. For instance, the output may be that "yes" the patient has delirium or "no" the patient does not have delirium. The output may also be that "yes" the patient is likely to develop delirium or "no" the patient is not likely to develop delirium.

The indication of the presence, absence, or likelihood of the subsequent development of delirium may be a non-binary indication. For example, the indication may be a percentage risk indicia, *i.e.,* a 70% chance of subsequent development of delirium. The indication of the presence, absence, or likelihood of the subsequent development of delirium may be categorical. For example, the indication may be that the user has a "high", "medium", or "low" risk, and intermediary categories, such as "medium-high" or "medium-low". The indication may also be on an arbitrary scale, such as from 1-5, 1-10, etc.

Indications of the likelihood of a patient subsequently developing delirium may be based on percentage likelihoods. For example, an indication that a patient is likely to subsequently develop delirium may be based on a likelihood of more than 50%, more than 55%, more than 60%, more than 65%, more than 70%, more than 75%, more than 80%, more than 85%, more than 90%, or more than 95% that the patient will subsequently develop delirium.

The indications may be for any of the monitoring, screening, or predicting of delirium. The indications may also calculate and/or output a confidence score. For example, the indication may include a confidence score of 80% that the patient will develop delirium in a particular time period.

## Claims

1. A system for screening delirium patients for the presence of encephalopathy, comprising:
a. a handheld screening device comprising a housing;
b. two sensors configured to record one or more brain signals and generate one or more values for a number of occurrences of 3 Hz waves and a number of occurrences of 10 Hz waves over a period of time;
c. a processor; and
d. at least one module configured to:
i. perform spectral density analysis on the one or more values; and
ii. output data presenting an indication of the presence, absence, or likelihood of the subsequent development of encephalopathy based on a comparison of a value of a ratio of the number of occurrences of 3 Hz waves to the number of occurrences of 10 Hz waves to a threshold ratio value of 1.44, wherein the value of the ratio of the number of occurrences of 3 Hz waves to the number of occurrences of 10 Hz waves being greater than or equal to the threshold ratio value is an indication of the presence of encephalopathy and the value of the ratio of the number of occurrences of 3 Hz waves to the number of occurrences of 10 Hz waves being less than the threshold ratio value is an indication of the absence of encephalopathy.

2. The system of claim 1, wherein the one or more brain signals are electroencephalogram (EEG) signals.

3. The system of claim 1, wherein the system further comprises a ground lead.

4. The system of claim 1, wherein the housing comprises a display.

5. The system of claim 4, wherein the processor is disposed within the housing.

## Patentansprüche

1. System zum Screening von Delir-Patienten auf das Vorliegen von Enzephalopathie, umfassend:
a. eine in der Hand gehaltene Screening-Vorrichtung, die ein Gehäuse umfasst;
b. zwei Sensoren, ausgelegt zum Aufzeichnen eines oder mehrerer Gehirnssignale und Erzeugen eines oder mehrerer Werte für eine Anzahl des Auftretens von 3-Hz-Wellen und eine Anzahl des Auftretens von 10-Hz-Wellen über einen Zeitraum;
c. einen Prozessor; und
d. mindestens ein Modul, das zu Folgendem ausgelegt ist:
i. Durchführen einer Spektraldichteanalyse an dem einen oder den mehreren Werten; und
ii. Ausgeben von Daten, die eine Angabe des Vorliegens, des Nichtvorliegens oder der Wahrscheinlichkeit einer späteren Entwicklung von Enzephalopathie basierend auf einem Vergleich eines Werts eines Verhältnisses der Anzahl des Auftretens von 3-Hz-Wellen zu der Anzahl des Auftretens von 10-Hz-Wellen mit einem Schwellenverhältniswert von 1,44 präsentiert, wobei die Tatsache, dass der Wert des Verhältnisses der Anzahl des Auftretens von 3-Hz-Wellen zu der Anzahl des Auftretens von 10-Hz-Wellen größer oder gleich dem Schwellenverhältniswert ist, auf das Vorliegen von Enzephalopathie hindeutet und die Tatsache, dass der Wert des Verhältnisses der Anzahl des Auftretens von 3-Hz-Wellen zu der Anzahl des Auftretens von 10-Hz-Wellen kleiner oder gleich dem Schwellenverhältniswert ist, auf das Nichtvorliegen von Enzephalopathie hindeutet.

2. System nach Anspruch 1, wobei das eine oder die mehreren Gehirnssignale Elektroenzephalogramm(EEG)-Signale sind.

3. System nach Anspruch 1, wobei das System ferner eine Masseleitung umfasst.

4. System nach Anspruch 1, wobei das Gehäuse eine Anzeige umfasst.

5. System nach Anspruch 4, wobei der Prozessor in dem Gehäuse angeordnet ist.

## Revendications

1. Système de dépistage de la présence d'encéphalopathie chez des patients atteints de délire, comprenant :
a. un dispositif de dépistage à main comprenant un boîtier ;
b. deux capteurs configurés pour enregistrer un ou plusieurs signaux cérébraux et générer une ou plusieurs valeurs pour un nombre d'occurrences d'ondes à 3 Hz et un nombre d'occurrences d'ondes à 10 Hz sur un laps de temps ;
c. un processeur ; et
d. au moins un module configuré pour :
i. effectuer une analyse de densité spectrale sur la ou les valeurs ; et
ii. délivrer des données présentant une indication de la présence, l'absence ou la probabilité du développement ultérieur d'encéphalopathie sur la base d'une comparaison d'une valeur d'un rapport entre le nombre d'occurrences d'ondes à 3 Hz et le nombre d'occurrences d'ondes à 10 Hz à une valeur de rapport seuil de 1,44, dans lequel une valeur du rapport entre le nombre d'occurrences d'ondes à 3 Hz et le nombre d'occurrences d'ondes à 10 Hz supérieure ou égale à la valeur de rapport seuil est une indication de la présence d'encéphalopathie et une valeur du rapport entre le nombre d'occurrences d'ondes à 3 Hz et le nombre d'occurrences d'ondes à 10 Hz inférieure à la valeur de rapport seuil est une indication de l'absence d'encéphalopathie.

2. Système de la revendication 1, dans lequel le ou les signaux cérébraux sont des signaux d'électroencéphalogramme (EEG).

3. Système de la revendication 1, le système comprenant en outre un fil de masse.

4. Système de la revendication 1, dans lequel le boîtier comprend un écran.

5. Système de la revendication 4, dans lequel le processeur est disposé à l'intérieur du boîtier.
